# EUROPEAN PATENT APPLICATION

(11) **EP 3 779 492 A1**
(43) Date of publication of application: **17.02.2021**
(21) Application number: 19191650.1
(22) Date of filing: 14.08.2019
(51) Int. Cl.: G01R 33/28, A61B 5/06, G01N 21/88, G01N 21/94

(54) **OPTICAL DETECTION OF FOREIGN METAL OBJECTS ON SUBJECTS BEFORE UNDERGOING MAGNETIC RESONANCE IMAGING**

(71) Applicant: Koninklijke Philips N.V., 5656 AG Eindhoven (NL)
(72) Inventor: CHAUDHURY, Sudipta, 5656 AE Eindhoven (NL); SISODIA, Rajendra, Singh, 5656 AE Eindhoven (NL); WEISS, Steffen, 5656 AE Eindhoven (NL)
(74) Representative: de Haan, Poul Erik

(57) **Abstract**

Provided is an optical metal detector device (10) for preparing magnetic resonance imaging, MRI, comprising: a light source (11) configured to emit light to illuminate a subject (S), a camera (12) configured to be positioned in multiple different positions relative to the subject, to capture a series of images of the illuminated subject and to provide assigned image signals, a linear polarizer (13) configured to be arranged in at least a first angular position and a second angular position relative to the subject, the first angular position and the second angular position are different to each other, and to polarize the light emitted by the light source or to polarize the image captured by the camera, and a data processing means (14) configured to obtain at least the image signals from the camera, wherein the data processing means is further configured to combine the image signals associated with the first angular position and the second angular position of the linear polarizer and the image signals associated with one or more of the multiple different positions of the camera to obtain a combined signal and to compare the combined signal with a threshold indicating a quantity of foreign metal objects on the subject.

## Description

### FIELD OF THE INVENTION

The present invention relates to optical metal detection, particularly for preparing magnetic resonance imaging, MRI. In particular, the invention relates to an optical metal detector device, a method of optically detecting metal, a computer program product and a computer-readable medium or data carrier.

### BACKGROUND OF THE INVENTION

Safety, in particular patient safety, is paramount during any imaging of a subject. In particular, in magnetic resonance imaging, MRI, where magnetic fields are used, metal objects on or inside the subject, for example, adhering to the skin or stuck in the skin or even embedded deeper in the tissue, may cause injury and/or harm if exposed to the magnetic fields. For example, presence of metal objects on or inside the subject during MRI may harm patients for two reasons, namely because some metals are Ferro-magnetic and experience strong forces caused by the powerful magnetic field, and because metals are often conductive and may heat up during MRI due to currents induced either by magnetic resonance, MR, gradient coils radio frequency, RF, coils used with MR.

Therefore, safety questions may be asked to the subject in advance related to any metal objects/implants/jewelry the subject may have. These metal objects, which are recognized by asking questions, can then be considered during MRI or, if the risk for the subject is considered to be too high, the MRI can be aborted. The risk may be caused by the mere presence of metal objects, their material, their size, their position on or in the subject, etc.

Further, metal detectors may be used to detect metal objects on or in a subject. These metal detectors may be used in the form of either a walk-through metal detector or a handheld metal detector. Such metal detectors may be able to detect comparatively large metal objects, but are not able to reliably detect small or fine metal objects. However, even small or fine metal objects may cause injury if exposed to a magnetic field. Also, such metal detectors are not well-suited for an autonomous imaging system, where there is little to no human invention and therefore even small metal objects should be reliably detectable.

### SUMMARY OF THE INVENTION

There may, therefore, be a need to improve detection of metal objects in preparation of magnetic resonance, MR, and particularly of magnetic resonance imaging, MRI. The object of the present invention is solved by the subject-matter of the appended independent claims, wherein further embodiments are incorporated in the dependent claims.

According to a first aspect, there is provided an optical metal detector device applicable for preparing magnetic resonance imaging, MRI. The optical metal detector device comprises:
a light source configured to emit light to illuminate a subject,
a camera configured to be positioned in multiple different positions relative to the subject, to capture a series of images of the illuminated subject and to provide assigned image signals,
a linear polarizer configured to be arranged in at least a first angular position and a second angular position relative to the subject, the first angular position and the second angular position are different to each other, and to polarize the light emitted by the light source or to polarize the image captured by the camera, and
a data processing means configured to obtain at least the image signals from the camera,
wherein the data processing means is further configured to combine the image signals associated with the first angular position and the second angular position of the linear polarizer and the image signals associated with one or more of the multiple different positions of the camera to obtain a combined signal and to compare the combined signal with a threshold indicating a quantity of foreign metal objects on the subject.

The proposed optical metal detector device may be particularly applicable to MRI preparation in either a patient preparation room or before an MR scanning room, where accordingly the camera, the light, the linear polarizer and/or the data processing means may be located. The optical metal detector device may be coupled to an MRI device and may be further configured to directly or indirectly control the MRI device. An exemplary field of application may be the examination of subjects working in the metal processing industry. Industrial workers working in metal, welding, mining, and construction industry may be at risk of deposition of fine metal dust on or in their skin and eyes, which may not be detected or detrimental in everyday life but become a threat during MR imaging. Since this group of people is also prone to accidents, the need of MR scans occurs more often. Of course, the proposed optical metal detector device may also be applicable to patients or subjects not particularly working in the metal processing industry, but potentially having metal objects.

Preferably, the subject may be a human or animal patient, where the metal objects to be detected may be on the skin, partially stuck in the skin or may also be enclosed in the skin or underlying tissue.

The light source may be further configured to illuminate the subject with light of a particular wavelength that may be different to a wavelength of an ambient light in the surroundings of the optical metal detector device. The light source may comprise one or more light emitters, such as LEDs or the like.

The camera may be provided as a camera system comprising one or more cameras, which may be coupled to the data processing means for data communication. The camera may, for example, have an actuator configured to move the camera, for example, by shifting, pivoting etc., to adjust multiple various relative positions of the camera to the subject. For example, a first image may be captured in a first position, a second image in a second position, a third image in a third position of the camera, etc.

The linear polarizer may be moved step by step to at least two, preferably to more than two, various fixed angular positions between 0 and 90 degrees. The steps may be in angles such as 5, 15, 20, 25, 30, 35, 40 etc. degree steps, wherein at a starting angle of 0° a first image may be taken and for example at an angle of 30° a second image, at an angle of 45° a third image etc. may be taken. In particular, the linear polarizer may be rotatable mounted, so that the various angular positions may be reached by rotating. It is noted that the higher the number of different angular positions of the linear polarizer and the resulting number of images from the camera is, the more accurate the detection result may be. Thereby, the largest reflecting surface of each particle contributes to a maximum intensity at a particular polarizer angle, whereas e.g. skin pigments maintain a nearly uniform reflective property across all polarization angles.

It is noted that the camera may capture multiple sets of images with different positions of the camera and for each camera position also different angular positions of the linear polarizer. The images of these sets may then be combined to, in the combined signal, distinguish between areas of the subject in which more or less light reflections have occurred. By observing or detecting the subject from different positions, which can result in different angles between the camera and the subject, the possibility of recognizing a light-reflecting surface of a metal object to be detected is increased, i.e. the sensitivity of the measurement is increased.

The data processing means may be any suitable data processor, computer, distributed computer system or the like. The function of combining the signals to the combined signal may be implemented as software or hardware. The data processing means may be further configured to quantify an amount of metal particles and/or metal dust and to identify a risk for the subject as a function of the quantified amount. The data processing means may configured to apply appropriate filters to detect metal objects, preferably even fine metal grains.

An effect of this metal detector device is that even small and/or fine metal objects, such as metal particle, metal dust, or the like, may be detected reliably and even autonomously and/or highly or fully automated. The metal detector device may be able to differentiate between similar intensity, such as moisture/perspiration, and metal particles based on the multiple polarizer angles. Also, it allows a repetitive polarized image acquisition and uses an intensity curve instead of single data points. Overall, a particularly high signal-to-noise ratio, snr, may be achieved by combining the signals to the combined signal.

According to an embodiment, combining the image signals may comprise a convolution operation.

In this context, the convolution operation may be understood as a mathematical operation on two or more signals, which may be expressed as functions, to produce a third signal, namely the combined signal, which may be expressed as a function. The convolution operation may be carried out by a data processing means, such as a processor of a computer. The combined signal may have a shape or course of one of the signals that is modified by the other signal. For example, intensity histograms obtained from the captured images may be convoluted with each other to generate an image devoid of facial features. The convolution of multiple polarizer angles may improve the snr. For example, the highest peaks representing the brightest refection points at a particular pixel in the combined images. The scattered and/or absorbed components may be subjected to a very minimal value post convolution. A reconstruction of the original image from the convoluted signals may have to be thresholded to filter out any other objects other than highest reflecting points.

In an embodiment, the combined signal represents one or more portions of the subject in which light-reflecting metal objects are present.

Thus, the intensity of light-reflections may be detected and/or quantified for various different portions of the subject.

According to an embodiment, the data processing means may be further configured to generate an alarm signal if the threshold is violated.

Thus, metal particles and/or metal dust may be quantified, wherein a risk for the subject may be estimated on the basis of the threshold value. A high accumulated amount of particles may present a higher risk than a low accumulated amount. If the amount and/or the risk is considered too high, the alarm signal may be generated. As a result of the alarm signal, MRI may be postponed or cancelled to avoid injuries caused by the metal objects exposed to the magnet fields.

In an embodiment, the data processing means may be further configured to assign various thresholds for foreign metal objects specifically to various body portions of the subject.

Thus, it may be distinguish between the various body portions of the subject, where some portions may have a more sensitive tissue and some portions may have a less sensitive tissue. Accordingly, the threshold may be set based on the specific body portion, wherein the threshold may be higher for less sensitive tissue and lower for more sensitive tissue. For example, especially the tissue in or near the eyes may be considered more sensitive, whereas a forearm is considered less sensitive, or the like.

According to an embodiment, the light source may be configured to be positioned in multiple different positions relative to the subject and the data processing means may be further configured to combine image signals obtained for one or more of the different positions of the light source.

Thus, the detection sensitivity and/or the snr may be further improved. The illumination of the subject from different positions, which may result in different angles between light source and subject, increases the possibility to detect a light-reflecting surface of a metal object to be detected.

In an embodiment, the linear polarizer may be arranged in a light beam path between the light source and the subject or between the subject and the camera.

Thus, a good polarization may be achieved, which improves the detection of a light-reflecting surface of a metal object to be detected.

In at least some embodiments, the linear polarizer may be arranged in the light beam path between the light source and the subject. Thus, this may be result in the same modulation of light reflected from metal objects with rotation angle. However, a reduced snr may be achieved because light scattering in the skin partially destroys the polarization of any light both on the way from skin surface to the metal particle and on the way back, and if unpolarized light is used for illumination, then no loss of polarization may happen on the way to the metal particle, so that there may be no loss of snr, and vice versa, if polarized light is used for illumination, then part of that polarization is lost by scattering already on the way to the metal particle so that there may be loss of snr.

According to an embodiment, a bandwidth or wavelength of the light emitted by the light source is from near-infrared to ultraviolet.

The bandwidth or wavelength may be selected and/or varied based on a depth of metal objects to be detected relative to a surface of the subject. Penetration depth of light in human skin increases with wavelength. Near-infrared may be specified as IR-A or IR-B, having a wavelength from about 780 nm to about 3000 nm, wherein IR-A is from about 780 nm to about 1400 nm and IR-B is from about 1400 nm to about 3000 nm. Ultraviolet may be specified as UV-A, UV-B or UV-C, having a wavelength from about 100 to about 380 nm, wherein UV-A is from about 315 nm to about 380 nm, UV-B is from about 280 nm to about 315 nm and UV-C is from about 100 to about 280 nm.

Thus, the bandwidth or wavelength may be selected to penetrate the subject S, wherein a penetration depth of the light depends on the selected bandwidth or wavelength.

In an embodiment, a band-pass filter having a passband that transmits the light emitted by the light source and that at least partially blocks ambient light is applied to the camera.

Thus, this allows the ambient light, which may be present in the surroundings of the metal detector device to be suppressed so that the detection sensitivity may be increased.

According to a second aspect, there is provided a method of detecting foreign metal objects on a subject, particularly for preparing magnetic resonance imaging, MRI. The method comprises:
illuminating, by a light source, the subject with light,
moving a linear polarizer from at least a first angular position to a second angular position relative to the subject,
moving a camera from at least a first position to a second position relative to the subject,
capturing, by the camera, a series of images of the illuminated subject, at least a first image of the series associated with the first position, a second image of the series associated with the second position of the linear polarizer, a third image of the series associated with the first position of the camera and a fourth image of the series associated with the second position of the camera, and
combining, by a data processing means, the images obtained by the camera to obtain a combined signal, and
comparing the combined signal with a threshold indicating a quantity of the foreign metal objects on the subject.

The method may be carried out by use of the optical metal detector device according to the first aspect. Accordingly, the method may be adjusted to any one of the preceding embodiments described in accordance with the metal detector device.

The illumination of the subject with light of the light source may cause a surface of the metal object to be detected to reflect the light. The largest light-reflecting surface of each metal object may contribute to a maximum intensity at a particular linear polarizer angular position. A modulation of reflectance with polarizing angle becomes large for large incident angles. Metallic particles that are produced during machining may have one large flat surface, which in general is not angulated such that it reflects light from the light source to the camera. To increase detection sensitivity, it is proposed to acquire multiple sets of images with different positions, i.e. glancing angles, of the camera. This may increase the probability that the dominant surface of a metal particle may be seen under the glancing angle for one of the positions.

According to an embodiment, the metal objects to be detected may be present as particles or dust.

Thus, the method may be used to detect even small and/or fine metal objects, which cannot be detected by conventional metal detectors or which cannot be assigned to a specific body region of the subject.

In an embodiment, the threshold, or multiple thresholds each assigned to a specific body portion of the subject, may be set based on at least one anatomical feature of the subject, and the threshold may be higher for less sensitive tissue and lower for more sensitive tissue.

Thus, an alarm that indicates presence of metal objects in a size, amount or quantity that is considered risky for the subject when exposed to magnetic fields during MRI may be generated based on the location of the detected metal object. The alarm may comprise a signal configured to control the MRI, so as to prevent start of MRI or to interrupt MRI immediately.

According to an embodiment, a wavelength of the light emitted by the light source may be selected as a function of a detection depth of the foreign metal objects with reference to an outer surface of the subject.

For example, the wavelength may be varied during detection or operation of the optical metal detector device of the first aspect. The penetration depth of light in human skin may increase with wavelength.

According to a third aspect, there is provided a computer program product for detecting metal objects on a subject, comprising instructions which, when being executed by a computer, cause the computer to carry out the method steps of the second aspect.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

According to a fourth aspect, there is provided a computer-readable medium or data carrier comprising or carrying the computer program product of the third aspect.

The computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems, e.g. in form of a data stream or the like. According to a further exemplary embodiment of the present invention, a medium for making a computer program element available for downloading is provided, which computer program element is arranged to perform a method according to one of the previously described embodiments of the invention.

According to a further aspect, there is provided an MRI system, comprising an MRI device and the optical metal detector device of the first aspect. The optical metal detector device may optionally be configured to generate an alarm signal if an amount or quantity of metal objects are detected that is considered to be risky for the subject during MRI. Upon the alarm signal, the MRI may be postponed, abandoned or cancelled. If no alarm signal is generated, either no or at most an uncritical amount of metal objects have been detected, and the MRI may be carried out.

These and other aspects of the present invention will become apparent from and elucidated with reference to the embodiments described hereinafter.

### BRIEF DESCRIPTION OF THE DRAWINGS

Exemplary embodiments of the invention will be described in the following drawings.
Fig 1 shows in a schematic perspective overview an MRI system comprising an MRI device and an optical metal detector device according to an exemplary embodiment of the invention.
Fig. 2 shows in a schematic side view an optical metal detector device according to an exemplary embodiment of the invention separate from an MRI device.
Fig. 3 shows different angular positions of a linear polarizer and resulting images of a foreign metal object taken at the different angular positions of the linear polarizer by use of a camera of an optical metal detector device according to an exemplary embodiment of the invention.
Fig. 4 shows exemplary cropped parts of an image signal of a metal object taken at the different angular positions of the linear polarizer by use of a camera of an optical metal detector device according to an exemplary embodiment of the invention.
Fig. 5 shows a flow chart of a method of detecting foreign metal objects in or on a subject.

### DETAILED DESCRIPTION OF EMBODIMENTS

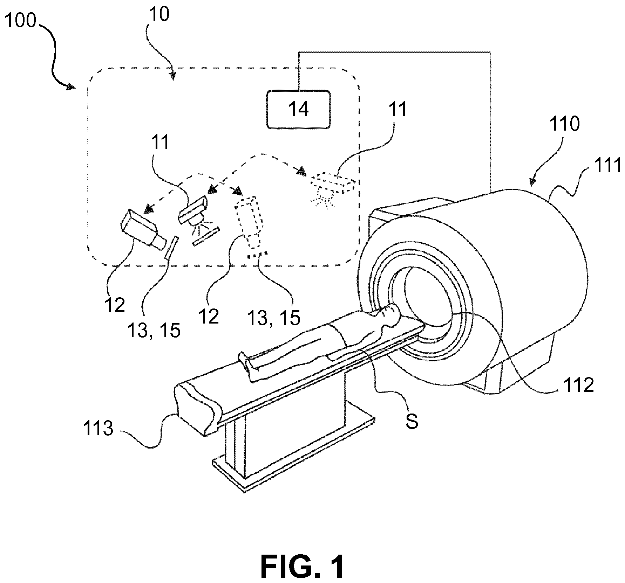

Fig. 1 shows in a schematic perspective overview a magnetic resonance imaging, MRI, system 100 comprising a magnetic resonance imaging, MRI, device 110 and an optical metal detector device 10 according to an exemplary embodiment. The MRI device 110 and the optical metal detector device 10 are operatively connected to each other, so as to control the MRI device 110 based on output control data of the optical metal detector device 10. In particular, the MRI device 110 may be configured to be started or to be switched off on basis of output control data of the optical metal detector device 10. Accordingly, in this exemplarily embodiment as shown in Fig. 1, the MRI device 110 and the optical metal detector device 10 are located within the same room which exemplarily is a scanning room. A patient or subject S is to be examined for undesired metal objects by using the optical metal detector device 10 before the MRI is performed. By way of example, the subject S may have metal objects to be detected which are present as particles or dust.

The MRI device 110 comprises a magnetic unit 111 that defines a bore 112 into which the patient or subject S can be moved by means of a subject supporting couch 113. For better illustration, in Fig. 1, the subject S is placed on the couch 113.

The optical metal detector device 10 comprises at least one light source 11 configured to emit light in a defined and/or predetermined and/or adjustable wavelength to illuminate the subject S. For example, the light source 11 may be configured to emit light from near-infrared to ultraviolet, wherein the wavelength may be varied to provide different penetration depths of the emitted light with respect to a surface or skin of the subject S, which penetration depths depend on the wavelength used. Preferably, the wavelength used is different to a wavelength of an ambient light in the surroundings of the optical metal detector device 10. The light source 11 may be configured to be positioned in multiple different positions relative to the subject S, which different positions of the light source 11 are indicated in Fig. 1 by showing the light source 11 in solid lines in a first position and in dashed lines in a second position. It is understood that the second position may be achieved from the first position by moving, pivoting etc., wherein to perform the movement, the optical metal detector device 10 may have one or more actuators that may be controlled electronically. Thus, the subject S may be illuminated from different positions and/or at different angles, so that the reflection intensity of illuminated metal objects changes.

Further, the optical metal detector device 10 comprises at least one camera 12 which may be adapted to acquire multiple images of the subject S. In operation of the optical metal detector device 10, the camera 12 is configured to capture images of the subject S being illuminated by the light of the light source 11. The camera 12 may be configured to be positioned in multiple different positions relative to the subject S, to capture a series of images of the illuminated subject S and to provide assigned image data and/or signals. The multiple different positions of the camera 12 are indicated in Fig. 1 by showing the camera 12 in solid lines in a first position and in dashed lines in a second position. It is understood that the second position may be achieved from the first position by moving, pivoting etc., wherein to perform the movement, the optical metal detector device 10 may comprise one or more actuators that may be controlled electronically. Thus, the subject S may be detected from different positions and/or at different angles, so that the reflection intensity of illuminated metal objects changes.

Further, the optical metal detector device 10 comprises at least one linear polarizer 13, which may be rotatable with respect to the camera 12 and/or the light source 11, in particular around an axis of view of the camera 12 and/or around an axis corresponding to a light beam of the light source 11. In particular, the linear polarizer 13 is configured to be arranged in at least a first angular position and a second angular position, relative to the subject S, wherein the first angular position and the second angular position are different to each other. Further, the linear polarizer 13 may be configured to polarize the light emitted by the light source 11 or to polarize the image captured by the camera 12. By way of example, in Fig. 1, the linear polarizer 13 is arranged in the front of the camera 12, so as to be located between the camera 12 and the subject S. In at least some embodiments, however, the linear polarizer 13 may additionally or alternatively be arranged in a light beam path between the light source 11 and the subject S. It is understood that the different may be achieved from the first position by rotating the linear polarizer 13, wherein to perform the movement, the optical metal detector device 10 may comprise one or more actuators that may be controlled electronically.

Further, the optical metal detector device 10 comprises a data processing means 14 configured to obtain at least the image signals from the camera 12. It may be provided as a processor, a computing means etc. The data processing means 14 may be operatively connected to the camera 12, and may be further operatively connected to light source 11 and/or the linear polarizer 13, for providing control signals for movement and/or for obtaining feedback signals regarding a current position or alignment. The data processing means 14 may be further configured to combine the image signals associated with the first angular position and the second angular position of the linear polarizer 13 and the image signals associated with one or more of the multiple different positions of the camera 12 to obtain a combined signal and to compare the combined signal with a threshold indicating a quantity of foreign metal objects on the subject S. The threshold may be assigned to a specific body portion of the subject S, and may be set based on at least one anatomical feature of the subject S, wherein the threshold may be higher for less sensitive tissue and lower for more sensitive tissue. By way of example, an eye region may be regarded as a more sensitive tissue, whereas an arm, a leg or the like may be regarded as less sensitive tissue. The threshold may indicate a quantity or density of e.g. metal dust, with optional consideration of the type of tissue and/or metal object, the penetration depth, duration of the planned MRI scan, magnet field intensity etc. The data processing means 14 is further configured to assign various thresholds for foreign metal objects specifically to various body portions of the subject S. Further, the data processing means 14 may be configured to generate an alarm signal if the one or more thresholds are violated. Also, the data processing means 14 may be configured to combine the images obtained by the camera 12 to obtain a combined image signal. Further, the data processing means 14 may be configured to compare the combined image signal with the one or more thresholds above.

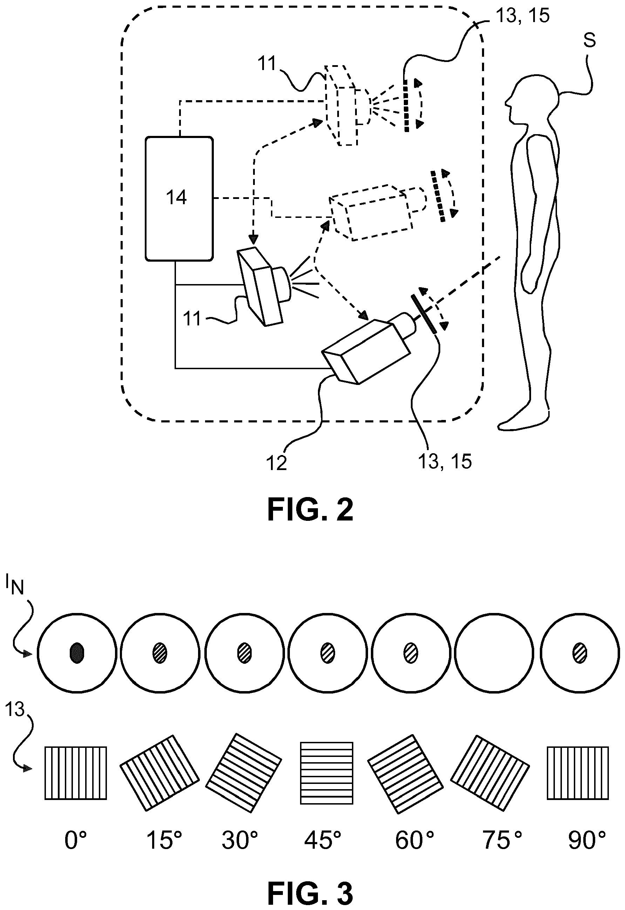

Fig. 2 shows a schematic side overview of the optical metal detector device 10 according to an exemplary embodiment, where it is separate from the MRI device 110. In this embodiment, the optical metal detector device 10 may be located in a separate preparation room instead of the MR scanning room. However, the functionality of this embodiment is preferably the same as described above with regard to Fig. 1. It shows, however, that the optical metal detector device 10 does not necessarily depends on the presence of the MRI device 110. As denoted by reference signs 15, in at least some embodiments, a band-pass filter having a passband that transmits the light emitted by the light source and that at least partially blocks ambient light may applied to the camera 12.

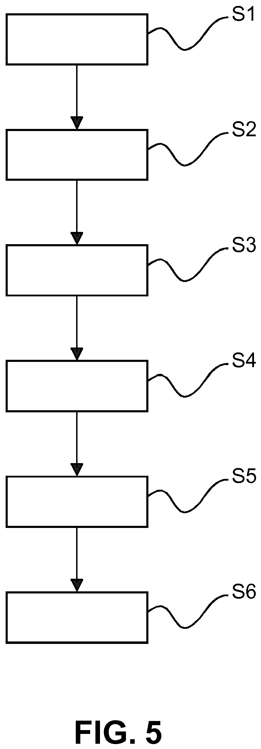

Fig. 3 shows different angular positions of the linear polarizer 13 and resulting captured images I_{N} of the optical metal detector device 10 according to an exemplary embodiment of the invention. Fig. 3 comprises three rows, whereby in a first row, here upper row, seven different images I₁ to I₆ taken by the camera 12 are contained, which images are assigned in each case to an angular position of the linear polarizer 13. In the first row of Fig. 3, black ovals of varying intensity indicate that the detectable intensity of reflection caused by the illumination of the metal object, which is denoted by M, or subject S by the light source 11 changes with the angular position of the linear polarizer 13. In a second, middle row the linear polarizer 13 is shown in the seven different angular positions, namely at 0° as start position, at 15°, 30°, 45°, 60°, 75° as intermediate positions and at 90° as end position. As can be seen in Fig. 3, by way of example, at 0° the intensity may be maximum, wherein in the intermediate position the intensity decreases. The intermediate positions are reached by turning the linear polarizer 13 by e.g. 30°, wherein other angular steps are possible. The third, lowest row of Fig. 3 designates the seven different angular positions of the linear polarizer 13 with 0° as start position, and 15°, 30°, 45°, 60°, 75° as intermediate positions and 90° as end position. As will be described below, the sequence of images I_{N} when convoluted will highlight the portions with metal dust with preferably a Gaussian enhancement (see e.g. Fig. 5). The largest reflecting surface of each metal object M may contribute to a maximum intensity at a particular linear polarizer angle, whereas the skin pigments maintain a nearly uniform reflective property across all polarization angles. These intensity histograms may be convoluted with each other to generate an image devoid of e.g. facial features.

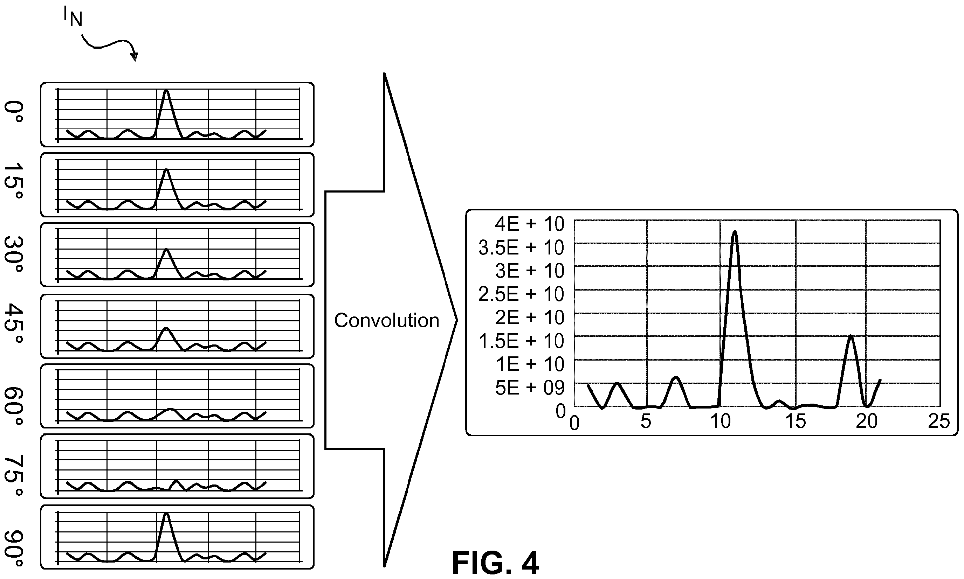

Fig. 4 shows exemplary cropped parts of an image signal of a metal object M taken at the different angular positions of the linear polarizer 13 by use of the camera 12 of the optical metal detector device 10 according to an exemplary embodiment of the invention. On the left are the several different images I_{N} known from Fig. 3. In the middle the combining operation, which may be a convolution operation, of these images I_{N} is indicated by an arrow. The combining operation may be performed by use of the data processing means 14. On the right the resulting combined, e.g. convoluted signal, is shown. The convolution of images taken at multiple polarizer angles increases the Signal to Noise Ratio. The highest peaks representing the brightest refection points at a particular pixel in the rotationally combined images. The scattered and/or absorbed components are subjected to a very minimal value post convolution. A reconstruction of the original image from the convoluted signals would have to be thresholded to filter out any other objects other than highest reflecting points.

Fig. 5 shows a flow chart of a method of detecting foreign metal objects on a subject S. In a step S1, the subject S is illuminated with light by the light source 11.

In a step S2, the linear polarizer 13 is moved from at least a first angular position to a second angular position relative to the subject S.

In a step S3, the camera 12 is moved from at least a first position to a second position relative to the subject (S). It is noted that steps S2 and S3 may also be performed in any different order.

In a step S4, a series of images of the illuminated subject S is captured by the camera 12, wherein at least a first image of the series associated with the first position of the linear polarizer 13 and a second image of the series associated with the second position of the linear polarizer 13, a third image of the series associated with the first position of the camera 12 and a fourth image of the series associated with the second position of the camera 12. It is noted that the first to fourth images may be obtained in any different order.

In a step S5, the images obtained by the camera 12 are combined by the data processing means 14 to obtain a combined signal. The combining operation may comprise a convolution operation.

In a step S6, the combined signal is compared with a threshold indicating a quantity of the foreign metal objects m in or on the subject S. The comparison step may be performed by the data processing means 14.

It has to be noted that embodiments of the invention are described with reference to different subject matter. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 100: MRI system
- 110: MRI device
- 111: magnetic unit
- 112: bore
- 113: support table

- 10: optical metal detector device
- 11: light source
- 12: camera
- 13: linear polarizer
- 14: data processing means
- 15: band-pass filter

- M: metal object (e.g. particle, dust etc.)
- S: subject

## Claims

1. An optical metal detector device (10) for preparing magnetic resonance imaging, MRI, comprising:
a light source (11) configured to emit light to illuminate a subject (S),
a camera (12) configured to be positioned in multiple different positions relative to the subject, to capture a series of images of the illuminated subject and to provide assigned image signals,
a linear polarizer (13) configured to be arranged in at least a first angular position and a second angular position relative to the subject, the first angular position and the second angular position are different to each other, and to polarize the light emitted by the light source or to polarize the image captured by the camera, and
a data processing means (14) configured to obtain at least the image signals from the camera,
wherein the data processing means is further configured to combine the image signals associated with the first angular position and the second angular position of the linear polarizer and the image signals associated with one or more of the multiple different positions of the camera to obtain a combined signal and to compare the combined signal with a threshold indicating a quantity of foreign metal objects on the subject.

2. The optical metal detector device of claim 1, wherein
combining the image signals comprises a convolution operation.

3. The optical metal detector device of claim 1 or 2, wherein
the combined signal represents one or more portions of the subject in which light-reflecting metal objects are present.

4. The optical metal detector device of any one of the preceding claims, wherein
the data processing means (14) is further configured to generate an alarm signal if the threshold is violated.

5. The optical metal detector device of any one of the preceding claims, wherein
the data processing means (14) is further configured to assign various thresholds for foreign metal objects specifically to various body portions of the subject.

6. The optical metal detector device of any one of the preceding claims, wherein
the light source is configured to be positioned in multiple different positions relative to the subject, and
the data processing means is further configured to combine image signals obtained for one or more of the different positions of the light source.

7. The optical metal detector device of any one of the preceding claims, wherein
the linear polarizer is arranged in a light beam path between light source and subject or between subject and camera.

8. The optical metal detector device of any one of the preceding claims, wherein
a wavelength of the light emitted by the light source is from near-infrared to ultraviolet.

9. The optical metal detector device of any one of the preceding claims, wherein
a band-pass filter (15) having a passband that transmits the light emitted by the light source and that at least partially blocks ambient light is applied to the camera.

10. A method of detecting foreign metal objects on or in a subject (S), for preparing magnetic resonance imaging, MRI, comprising:
illuminating (S1), by a light source, the subject with light,
moving (S2) a linear polarizer from at least a first angular position to a second angular position relative to the subject (S),
moving (S3) a camera from at least a first position to a second position relative to the subject (S),
capturing (S4), by the camera (12), a series of images of the illuminated subject, at least a first image of the series associated with the first position, a second image of the series associated with the second position of the linear polarizer, a third image of the series associated with the first position of the camera and a fourth image of the series associated with the second position of the camera, and
combining (S5), by a data processing means, the images obtained by the camera to obtain a combined signal, and
comparing (S6) the combined signal with a threshold indicating a quantity of the foreign metal objects on the subject.

11. The method of claim 10, wherein
the metal objects to be detected are present as particles or dust.

12. The method of claim 10 or 11, wherein
the threshold is set based on at least one anatomical feature of the subject, and
the threshold is higher for less sensitive tissue and lower for more sensitive tissue.

13. The method of any one of claims 10 to 12, wherein
a wavelength of the light emitted by the light source is selected as a function of a detection depth of the foreign metal objects with reference to an outer surface of the subject.

14. A computer program product for detecting metal objects on a subject, comprising instructions which, when being executed by a computer, cause the computer to carry out the method steps of any one of claims 10 to 13.

15. A computer readable medium or data carrier comprising or carrying the computer program product of claim 14.
